# EUROPÄISCHE PATENTSCHRIFT

(11) **EP 2 427 541 B1**
(45) Veröffentlichungstag und Bekanntmachung des Hinweises auf die Patenterteilung: **07.12.2016**
(21) Anmeldenummer: 10718495.4
(22) Anmeldetag: 05.05.2010
(51) Int. Cl.: C12M 1/00, C12M 3/06

(54) **VORRICHTUNG ZUR DURCHFÜHRUNG PHOTOCHEMISCHER PROZESSE**
DEVICE FOR PERFORMING PHOTOCHEMICAL PROCESSES
DISPOSITIF POUR L'EXÉCUTION DE PROCESSUS PHOTOCHIMIQUES

(30) Priorität: 08.05.2009 DE 102009020527
(43) Veröffentlichungstag der Anmeldung: 14.03.2012
(73) Patentinhaber: Ehrfeld Mikrotechnik BTS GmbH, 55234 Wendelsheim (DE)
(72) Erfinder: SCHAEL, Frank, 64287 Darmstadt (DE); NAGY, Karoly, 52070 Aachen (DE)
(74) Vertreter: Lütjens, Henning
(86) Internationale Anmeldenummer: PCT/EP2010/002748
(87) Internationale Veröffentlichungsnummer: WO 2010/127841

(56) Entgegenhaltungen:
- WO-A1-2006/020177
- US-A1- 2008 044 887
- US-B2- 7 374 928
- CHOUL-GYUN LEE ET AL.: "High-Density Algal Photobioreactors Using Light-Emitting Diodes", BIOTECNOLOGY AND BIOENGINEERING, Bd. 44, 1994, Seiten 1161-1167, XP002608891,
- LEE ET AL.: "A laboratory scale air-lift helical photobioreactor to increase biomass output rate of photosysnthetic algal cultures", NEW PHYTOL., Bd. 116, 1990, Seiten 331-335, XP002608892,
- NEDBAL ET AL.: BIOTECNOLOGY AND BIOENGINEERING, Bd. 100, Nr. 5, 1. August 2008 (2008-08-01), Seiten 902-910, XP002608893,
- HAI ET AL.: "Axenic cultivation of anoxygenic phototrophic bacteria, cyanobacteria, and microalgae in a new closed tubular glass photobioreactor", APPL. MICROBIOL. BIOTECHNOL., Bd. 53, 2000, Seiten 383-389, XP002608894,
- WATANABE ET AL.: "Development of a photobioreactor incorporating Chlorella sp. for removal of CO2 in stack gas", ENERGY CONVERS. MAGMET., Bd. 38, Nr. Suppl., 1997, Seiten S499-S503, XP002608895,

## Beschreibung

Gegenstand der vorliegenden Erfindung ist eine Vorrichtung zur Durchführung photochemischer Prozesse im Mikromaßstab, sowie die Verwendung der erfindungsgemäßen Vorrichtung zur Kultivierung von Photosynthese-betreibenden Zellen und/oder Mikroorganismen.

Photochemische Reaktionen kommen unter anderem bei der technischen Synthese von chemischen Verbindungen z.B. auf den Gebieten der Pharmazeutika, Pflanzenschutzmittel, Riechstoffe und Vitamine zum Einsatz. Unter dem Begriff photochemische Reaktionen werden Reaktionen verstanden, die durch elektromagnetische Strahlung vorzugsweise im UV-Bereich bis sichtbaren Bereich initiiert und/oder aufrechterhalten werden.

Photobiotechnologische Prozesse spielen bei der Kultivierung von pflanzlichen Zellen und Pflanzen aber auch von Photosynthese-betreibenden Bakterien eine Rolle. Durch die Einstrahlung von elektromagnetischer Strahlung aus künstlichen Quellen oder in Form von Sonnenlicht werden Zellen oder Mikroorganismen, die Photosynthese betreiben, kultiviert.

Unter Kultivierung wird die Schaffung und Aufrechterhaltung von Bedingungen verstanden, die ein Wachstum und eine Vermehrung der Zellen und/oder Mikroorganismen gewährleisten.

Photochemische und/oder photobiotechnologische Prozesse finden auch bei der Inaktivierung von Mikroorganismen und Viren durch UV-Strahlung statt.

Bei den genannten Prozessen, die im Folgenden zusammenfassend als photochemische Prozesse bezeichnet werden, besteht eine Herausforderung darin, eine gleichmäßige Bestrahlung eines Mediums zu gewährleisten.

Die Leistungsfähigkeit technischer Apparate zur Durchführung photochemischer Prozesse wird häufig durch die Eindringtiefe der elektromagnetischen Strahlung in das zu bestrahlende Medium limitiert. Insbesondere bei biologischen Medien (z.B. Algenkulturen, Blut, Milch), die eine hohe Opazität aufweisen, ist die Eindringtiefe von elektromagnetischer Strahlung oftmals auf einen Bereich von wenigen Mikrometern unterhalb der Medienoberfläche beschränkt.

Weiterhin gilt es, Abschattungseffekte im Bestrahlungsraum zu vermeiden. Abschattungseffekte führen dazu, dass in den Apparaten entweder eine gute Durchmischung in der Bestrahlungszone gewährleistet werden muss und/oder das Medium solange im Kreislauf geführt werden muss, bis ein gewünschter Umsatzgrad erreicht ist.
Die Gefahr der ungleichmäßigen Bestrahlung ist dabei sehr hoch. Es besteht die Gefahr, dass Teile des Mediums eine zu hohe Strahlendosis und andere Teile des Mediums eine zu geringe Strahlendosis erhalten. Bei der Inaktivierung von Mikroorganismen und/oder Viren in biologischen Produkten wie beispielsweise Lebensmitteln oder Blutplasma besteht z.B. die Gefahr, dass die Teile, die eine zu hohe Strahlendosis erfahren, irreversibel geschädigt werden, während in den Teilen, die eine zu geringe Strahlendosis erfahren, keine vollständige Inaktivierung von Viren und/oder Mikroorganismen erfolgt.

Photochemische Prozesse werden daher in der Technik häufig in Fallfilmreaktoren durchgeführt, um die Ausbildung eines Filmes mit geringen optischen Schichtdicken zur Maximierung des bestrahlten Volumens und zur Minimierung von Abschattungseffekten der einfallenden Strahlung zu nutzen. Diese Reaktoren haben den Nachteil, dass die Schichtdicke in der Regel eine Funktion der Betriebsbedingungen wie Durchflussrate und Temperatur und von Stoffdaten wie der Viskosität ist, und sich kaum unabhängig einstellen lässt.

DE 102 22 214 A1 beschreibt beispielsweise einen Photobioreaktor, bei dem mehrere, durch lichtdurchlässige Wände getrennte Kompartimente mit Schichtdicken von 5 bis 30 mm ausgebildet werden, um eine verbesserte Lichtausnutzung und den parallelen Betrieb bei hohen bis geringen Lichtintensitäten zu erlauben. Da die optimale Lichtausnutzung von der Lichtabsorption der Zellsuspension und damit von der Zellzahl abhängt, variiert die Effizienz einer solchen Vorrichtung während der Wachstumsphase der Zellen.

GB 2118572 beschreibt einen Apparat zur Durchführung einer Photobioreaktion mit einer flüssigen Zellkultur. Der Apparat besteht aus einem lichtdurchlässigen Teil, in dem das Medium turbulent geführt wird, und ein Kopfreservoir. Zur Förderung dient eine Peristaltikpumpe oder eine durch Gaseintrag erzeugte Druckdifferenz. Die Anlage enthält beispielsweise 4.6 L Medium, wobei etwa 4 L durch den bestrahlbaren Teil fließen. Der Gasaustrag von inhibierendem Sauerstoff sowie der Eintrag von CO2 erfolgt in Flussrichtung hinter dem Reaktor in Steigleitungen, in denen eine Trennung von Gas-/Flüssigphase durchgeführt wird.
Die Apparatur ist für photochemische und photobiologische Prozesse, bei denen ein relativ schneller Gasverbrauch erfolgt nicht optimal, da hier kein Gas innerhalb der Bestrahlungszone aus der Gasphase in die Lösung nachgeliefert werden kann. Ferner erfolgt der Austrag von in der Bestrahlungszone gebildeten Gasen wie bei photobiologischen Prozessen beispielweise Sauerstoff nicht am Ort der Gaserzeugung, so dass eine wachstums-inhibierende Wirkung des Sauerstoffs im Reaktor nicht ausgeschlossen werden kann. Die Ausführung, bei der das Medium in der Apparatur durch reine Gaszugabe gefördert wird, ist nicht ideal, da wegen Veränderung der Medieneigenschaften während des Wachstums der Mikroorganismen Medieneigenschaften wie Viskosität verändert wird und daher die Flussgeschwindigkeit über die Wachstumsphasen nicht oder nur mit besonderem Aufwand konstant gehalten werden kann. Aufgrund der bei dieser Betriebsweise unvermeidbaren Schaumbildung wird in der Schrift auch Antischaummittel eingesetzt, was nicht für alle Anwendungen wünschenswert ist.

Die Mikroverfahrenstechnik bzw. Mikroreaktionstechnik ist in den letzten Jahren zunehmend zu einem wichtigen Hilfsmittel in der chemischen und Forschung und Entwicklung geworden. Ursache ist die Forderung des Marktes, in immer kürzeren Zeiten neue Produkte und verbesserte Prozesse zu entwickeln.

Die modulare Mikroverfahrenstechnik bietet die Möglichkeit, verschiedene Mikroprozessmodule nach einem Baukastenprinzip zu einer kompletten Produktionsanlage im Kleinstformat zusammenzufügen. Neben der daraus resultierenden hohen Flexibilität und der Reduzierung von Abfällen aufgrund der verminderten Mengen an Chemikalien, die für Experimente in Mikroreaktionsanlagen benötigt werden, weist die Mikroverfahrenstechnik unmittelbare Vorteile für die chemische Prozessführung auf: Mikrostrukturierte Apparate verfügen über ein sehr großes Verhältnis von Oberflächen zum Volumen. Aus diesem Grund lassen sich beispielsweise Wärme- und Stofftransportvorgänge deutlich intensivieren.

Das große Verhältnis von Oberflächen zum Volumen kann auch genutzt werden, um den Strahlungstransport in einer Reaktionslösung gegenüber herkömmlichen photochemischen Apparaten deutlich zu verbessern. Die Verhältnisse in herkömmlichen Anlagen für photochemische Umsetzungen führen beispielsweise häufig dazu, dass nur geringe Konzentrationen an Edukten eingesetzt werden können. Das ist partiell die Folge davon, dass die Dicke der bestrahlten Flüssigkeitsschicht nicht gut kontrolliert werden kann.

Umgekehrt bedingen die kleinen charakteristischen Dimensionen von mikrostruktruierten Apparaten im Bereich von typischerweise 1 bis 2000 µm neben den besonderen Vorteilen auch besondere Herausforderungen, die im Vergleich zur makroskaligen Anlagentechnik technisch andere Lösungen, insbesondere den Einsatz von abgestimmten multifunktionalen Systemen erfordern (s. beispielsweise V. Hessel, S. Hardt, H. Löwe, "Chemical Micro Processing Engineering", Vol. 1-2, Wiley-VCH, Weinheim, 2005).
Kleine Abmessungen bringen beispielsweise in der Regel hohe Scherbelastungen für die zu bestrahlenden Medien, wodurch Kultivierungen von Mikroorganismen schwierig und nur unter Beachtung einer Vielzahl von aufeinander abgestimmten Randbedingungen ermöglicht werden. Die Kombination von verschiedenen Kennzeichen der Apparate ist dabei Gegenstand experimenteller Untersuchungen und kann kaum vorausgesagt werden.

In H. Ehrich et al., Application of Microstructured Reactor Technology for the Photochemical Chlorination of Alkylaromatics, Chimia 56 (2002), S. 647 bis 653 ist der Einsatz eines Mikrofallfilmreaktors für die selektive Photochlorierung von Toluol-2,4-diisocyanat beschrieben. Ein entsprechender Mikrofallfilmreaktor ist auch in DE10162801A1 beschrieben. Durch ein Fenster erlaubt dieser Reaktor zwar die Einkopplung von Strahlung, nutzt aber nicht die volle einfallende Strahlungsmenge, da ein Teil konstruktionsbedingt abgeschattet wird. Ferner hat dieser Reaktor den Nachteil, dass die Verweil- und Bestrahlungszeit nicht über einen weiten Bereich kontrollierbar sind, weil bei dem Fallfilmprinzip immer die Gefahr besteht, dass der Film abreißt.

Kommerziell erhältliche Apparaturen zur Kultivierung Photosyntese-betreibender Zellen und Mikroorganismen basieren entweder auf rechteckigen Glasgefäßen, die nicht umgepumpt werden, oder bestrahlten Rohrwendeln mit Ausgleichsbehältern, in denen Sensoren untergebracht sind und der Stoffaustausch erfolgt. Hier sind die verfügbaren optischen Schichtdicken im Zentimeter-Bereich. DE29607285U1 beschreibt beispielsweise einen Photobioreaktor mit plattenförmiger Einrichtung zur Kultivierung von Photosynthese-betreibenden Mikroorganismen. Der Austausch von Gasen ist hier nur im Zwischenbehälter möglich.

DE4411486C1 beschreibt ein Verfahren zur Kultivierung und Fermentation von Mikroorganismen mit einem ultradünnen, filmartigen Medienstrom zwischen stoff- und lichtdurchlässigen Material z.B. aus PE-Folien. Der Kohlendioxid- und Sauerstoffaustausch erfolgt über die Folien. Die dünne Schicht mit dem 50- bis 500-fachen Durchmesser der Zellen wird z.B. über Viereckregner auf Spalte verteilt und fließt dann schwerkraftgetrieben durch den Spalt. Durch den Stoffdurchtritt durch eine Folie ist die Austauschgeschwindigkeit limitiert. Als Lichtquelle wird eine Na-Dampflampe verwendet. Die Umlaufgeschwindigkeit des Mediums ergibt sich aus der Fließgeschwindigkeit des Mediums durch den Spalt und wird nicht aktiv durch ein Förderorgan festgelegt.

C.-G. Lee und B.O. Palsson (High-density algal photobioreactors using light-emitting diodes, Biotechnology and Bioengineering, 44, 1161-1167 (1994)) beschreiben eine Apparatur zur Algenzüchtung mit LED-Beleuchtung in einem rechteckigen, durchströmten Glasgefäß und Medienaufbereitung durch Ultrafiltration. Hier wird insbesondere berichtet, dass bei Verringerung der Gefäßdicke die Zellzahldichte der Kultur gesteigert werden kann. Dies wird mit der besseren Strahlungsausnutzung und verminderten Abschattungseffekten begründet. Durch die Kombination der Strahler mit einem rechteckigen Gefäß ohne weitere Fluidführung kann allerdings die Reaktorfläche durch die spotartige Abstrahlcharakteristik nicht optimal ausgeleuchtet werden.
Während für die Algenkultivierung eine Reihe von Apparaturen bekannt sind und zum Teil in Großversuchen erprobt sind, besteht weiter Verbesserungsbedarf hinsichtlich einer Reihe von technischen Eigenschaften wie Ausnutzung und Art der Strahlungsquellen, Gaseintrag, pH-Stabilisierung, Kontrolle und Überwachung von Kultivierungsbedingungen, letztlich mit dem Ziel die Anlagen wirtschaftlicher betreiben zu können.

Das Dokument US2008/0044887 A1 beschreibt einen mikrofluidischer oder mesofluidischen mäanderförmigen und durch externe Leuchtdioden bestrahlten Photobioreaktor.

Ausgehend vom Stand der Technik stellt sich damit die Aufgabe, eine Vorrichtung zur Durchführung photochemischer Prozesse bereitzustellen, die einen hohen Wirkungsgrad hinsichtlich der eingestrahlten elektromagnetischen Strahlung aufweist. Die gesuchte Vorrichtung soll gleichzeitig eine wohldefinierte und gleichmäßige Bestrahlung eines Mediums gewährleisten. Insbesondere soll die Strahlungsdosis, die das bestrahlte Medium erfährt, einstellbar sein. Die gesuchte Vorrichtung soll sowohl kontinuierlich als auch im Batchbetrieb zu betreiben sein. Sie soll nach Möglichkeit modular aufgebaut und damit flexibel einsetzbar sein. Sie soll einfach in der Handhabung und kostengünstig sein. Sie soll die Durchführung photochemischer Prozesse unter wirtschaftlichem Bedingungen ermöglichen.
Erfindungsgemäß wird diese Aufgabe durch eine mikrostrukturierte Vorrichtung gemäß Anspruch 1 gelöst. Gegenstand der vorliegenden Erfindung ist daher eine Vorrichtung zur Durchführung photochemischer Prozesse, mindestens umfassend eine Bestrahlungszone, eine Quelle für elektromagnetische Strahlung und Mittel zur Förderung eines Mediums durch die Vorrichtung, dadurch gekennzeichnet, dass das Volumen der Bestrahlungszone mindestens dem 0,5-fachen des Vorrichtungsvolumens entspricht.

Unter dem Vorrichtungsvolumen wird das Volumen der Vorrichtung verstanden, das sich aus den Volumina der Bestrahlungszone, der Zu- und Ableitungen, des Mittels zur Förderung des Mediums und etwaiger weiterer Komponenten der Vorrichtung zusammensetzt. Vorlagenbehälter, aus denen Medien in die erfindungsgemäße Vorrichtung eingespeist werden, sowie Auffangbehälter zur Aufnahme von Produkten zählen hierbei nicht zum Vorrichtungsvolumen.
Eine erfindungsgemäße Vorrichtung mit einem bestrahlten Volumen, das mindestens genauso groß ist wie das unbestrahlte Volumen der Vorrichtung, hat den Vorteil, dass die Strahlungsenergie sehr effizient genutzt werden kann.

Als Quelle für elektromagnetische Strahlung eignen sich alle dem Fachmann bekannten Strahlungsquellen, die elektromagnetische Strahlung der gewünschten Wellenlänge oder des gewünschten Wellenlängenbereichs emittieren. Bevorzugt werden eine oder mehrere Leuchtdioden verwendet.

Eine Leuchtdiode ist ein elektronisches Halbleiter-Bauelement, bei dem durch Stromfluss in Durchlassrichtung der Diode die Emission elektromagnetischer Strahlung angeregt werden kann. Die emittierten Wellenlängen sind vom verwendeten Halbleitermaterial abhängig. Leuchtdioden haben gegenüber z.B. Glühlampen den Vorteil, dass sie keine Temperaturstrahler sind und somit das Medium oder Teile der Vorrichtung nicht unnötig erwärmen. Sie emittieren Licht in einem begrenzten Spektralbereich; das Licht ist nahezu monochrom. Die Effizienz ist hoch und Leuchtdioden erlauben damit einen gezielten und effizienten Einsatz der emittierten Photonen.

Bevorzugt sind in der erfindungsgemäßen Vorrichtung mehrere Leuchtdioden in einer planaren Fläche angeordnet, die parallel zur Bestrahlungszone angeordnet ist. In einer bevorzugten Ausführungsform befinden sich auf zwei gegenüberliegenden Seiten der vorzugsweise ebenfalls planaren Bestrahlungszone parallel zur Bestrahlungszone angeordnete Leuchtdioden. Durch diese zweiseitige Bestrahlung wird eine hohe Effizienz bei der Bestrahlung erzielt.

Die Bestrahlungszone der erfindungsgemäßen Vorrichtung umfasst einen oder mehrere Kanäle, in denen das zu bestrahlende Medium durch die Bestrahlungszone gefördert wird.
Ein Kanal kann beispielsweise einen halbrunden, rechteckigen, trapezförmigen oder dreieckigen Querschnitt aufweisen. Bevorzugt ist er rechteckig oder halbrund ausgeführt. Besonders bevorzugt ist er halbrund ausgeführt.

Ein Kanal zeichnet sich durch eine Tiefe im Bereich von 10 µm bis 2000 µm, besonders bevorzugt im Bereich von 500 µm bis 1000 µm aus. Eine solche Tiefe gewährleistet, dass auch bei opaken Medien das Medium in der Bestrahlungszone vollständig durchstrahlt wird.
Zur Vermeidung eines hohen Druckverlustes ist ein Kanal zwischen 10 mm und 50 mm, besonders bevorzugt zwischen 15 mm und breit 40 mm breit. Die genannten Kanalbreiten haben zudem den Vorteil, dass sie eine große Bestrahlungsfläche bereitstellen, so dass die Strahlungsenergie effizient ausgenutzt wird.

Die Kombination aus Volumenverhältnis der Bestrahlungszone zum Vorrichtungsvolumen in Verbindung mit den genannten Abmessungen und Geometrien führt überraschenderweise zu einer besonders effizienten Nutzung der eingesetzten Strahlung (Lichtausbeute).

Ein oder mehrere Kanäle können je nach zu beleuchtender Fläche und gewünschtem Anlagenvolumen gerade, mäanderförmig und/oder in Form von mehreren parallelen Strängen verlaufen. Bevorzugt ist eine Ausführungsform, bei der ein oder zwei parallele Kanäle mäanderförmig in der Bestrahlungszone verlaufen. Hierdurch kann eine einfache Anpassung an die Strahlcharakteristik der Strahlungsquelle bei minimalem Druckverlust erreicht werden.

Bei mäanderförmiger Strömungsführung weisen die Kanäle in den Umlenkstellen vorzugsweise Verjüngungen auf, die eine Erhöhung der Strömungsgeschwindigkeit an diesen Stellen und damit eine Verhinderung oder zumindest eine Verminderung von Ablagerungen bewirken. Bei entsprechender Ausführung können die Verjüngungen je nach Strömungsgeschwindigkeit auch als Abrisskante für Gasblasen dienen, die dadurch an dieser Stelle verkleinert werden.

Die Führung der Kanäle ist vorzugsweise auf die Anordnung der Strahlungsquelle in der Weise abgestimmt, dass der Abstrahlkegel der Strahlungsquelle bei dem entsprechenden Abstand optimal genutzt wird.

Durch gezielte Medienführung wird auch der Anteil der schlechter beleuchteten Stellen vermindert - insbesondere bei der Verwendung einer Strahlungsquelle umfassend mehrere Leuchtdioden, bei denen bauartbedingt zwischen zwei benachbarten Leuchtdioden Zwischenräume entstehen, in denen die Strahlungsintensität vermindert ist. Daher sind die Stege zwischen den Kanälen bevorzugt so gestaltet, dass möglichst wenig Medium in schlecht beleuchtete oder unbeleuchtete Zwischenräume geführt wird.

Alternative Strahlungsquellen wie insbesondere Excimerlampen, die in vielen verschiedenen Abmessungen herstellbar sind, oder Metalldampflampen können ebenfalls eingesetzt werden, zeigen aber aufgrund der Abstrahlcharakteristik, die häufig zusätzlich einen Reflektor erfordert, nicht ganz so hohe Energieausnutzung wie Leuchtdioden.

In einer bevorzugten Ausführungsform umfasst die Bestrahlungszone eine planare Reaktionszonenplatte, in der die Kanäle mit mikrotechnischen Fertigungsverfahren eingearbeitet sind. Durch eine Abdeckung werden die Kanäle in Richtung der Strahlungsquelle verschlossen. Die Abdeckung ist so angepasst, dass sie für die verwendete Strahlung eine ausreichende Transparenz aufweist.

Die Reaktionszonenplatte kann aus Metall wie z.B. Edelstahl, Hastelloy, Titan, Monel oder Kunststoffen wie z.B. perfluorierte Polymerverbindungen (PFTE) Glas oder Graphit gefertigt werden. Die Reaktionszonenplatte kann aus geeigneten Halbzeugen mit spanabhebenden Verfahren, aus Kunststoffen auch durch Spritzguss- oder Prägetechniken erzeugt werden.

Ebenso ist es denkbar, die Kanäle durch den Einsatz eines oder mehrerer Abstandshalter, beispielsweise aus Metall oder Kunststoff, die zwischen zwei Abdeckungen angeordnet sind, zu realisieren.

Als Materialien für die transparente Abdeckung können je nach Anforderungen an die Transmission für die eingesetzte Strahlung Quarzglas, Glas oder transparente Kunststoffe wie Plexiglas eingesetzt werden.

In die Bestrahlungszone sind vorzugsweise auch Temperierkanäle eingebracht, an die ein Thermofluid zur thermischen Kontrolle angeschlossen werden kann. Weiterhin ist mindestens ein Temperatursensor vorhanden, der eine Temperaturregelung des bestrahlten Mediums ermöglicht.

Die erfindungsgemäße Vorrichtung umfasst weiterhin Mittel zur Förderung des zu bestrahlenden Mediums durch die Bestrahlungszone. Durch dieses Fördermittel kann die Strömungsgeschwindigkeit und damit die Verweilzeit des Mediums in der Bestrahlungszone gezielt eingestellt werden. In Kombination mit einer veränderbaren Strahlungsintensität kann somit die Strahlungsdosis des zu bestrahlenden Mediums genau eingestellt werden. Als Mittel zur Förderung kann beispielsweise eine Pumpe (Peristaltik-, Zahnrad-, Membran-, Kolben- oder Kreiselpumpen) eingesetzt werden.

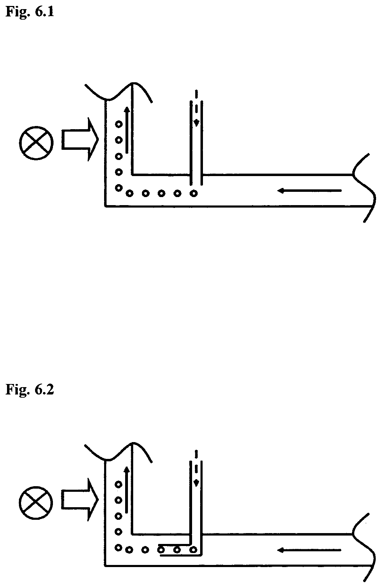

In einer besonders bevorzugten Ausführungsform ist die erfindungsgemäße Vorrichtung derart ausgestaltet, dass sich der Eingangsbereich der Bestrahlungszone in Bezug auf die Richtung der Schwerkraft unterhalb des Ausgangsbereichs der Bestrahlungszone befindet. Die hat zu Folge, dass das Medium entgegen der Schwerkraft durch die Bestrahlungszone gefördert werden muß. Diese Ausführungsform ist insbesondere dann vorteilhaft, wenn zusätzlich eine Gaseinspeisung im Eingangsbereich der Bestrahlungszone erfolgt, da es in diesem Fall zu einer ausgesprochen vorteilhaften Durchmischung des Mediums mit dem eingespeisten Gas kommt. Eine solche Ausführungsform ist in den Figuren 6.1 bis 6.3 beispielhaft abgebildet.

Es wurde nun überraschend gefunden, dass ein Photoreaktor mit Kanalabmessungen im Tiefenbereich von 10 bis 2000 µm und bei einer Breite im Bereich von 5 mm bis 200 mm, mit einer Gaszufuhr in der entgegen der Schwerkraftrichtung angebrachten Medienzufuhr, wobei feinverteilte Gasblasen in der Bestrahlungszone erzeugt werden, in Verbindung mit einer Peripherie wie Sensoren, Pumpeinrichtung, Gasabtrenneinrichtung, die nicht mehr als die Hälfte des Gesamtvolumens enthält, besonders gut geeignet ist, um photochemische Prozesse mit Gasen und Flüssigkeiten sowie photobiologische Prozesse wie die Kultivierung von phototropen Mikroorganismen durchzuführen.

In einer solchen Ausführungsform setzt die erfindungsgemäße Vorrichtung in einer mikrostrukturierten Bestrahlungszone des Photoreaktors die für die Durchführung der photochemischen oder photobiologischen Prozesses notwendiges Gas fein verteilt ein, um einerseits ein schnelles Nachliefern des Gases in die Flüssigphase, andererseits die bestrahlbare Fläche der Flüssigphase nicht zu stark zu beeinträchtigen.
Bei schnellen photochemischen Umsetzungen wird durch die Anwesenheit des Gases mit hoher Phasengrenzfläche die Konzentration des gelösten Gases und dessen Produkte als Reaktionspartner hoch gehalten. Bei photobiologischen Anwendungen sorgen die Gasblasen für eine zusätzliche Durchmischung und eine Verminderung von Ablagerungen in der Bestrahlungszone.
Die entgegen der Schwerkraftrichtung angebrachte Medienzu- und -abfuhr ermöglicht vorteilhafte Befüllung, Entlüftung der Bestrahlungszone sowie eine enge Verweilzeitverteilung des zu bestrahlenden Mediums.

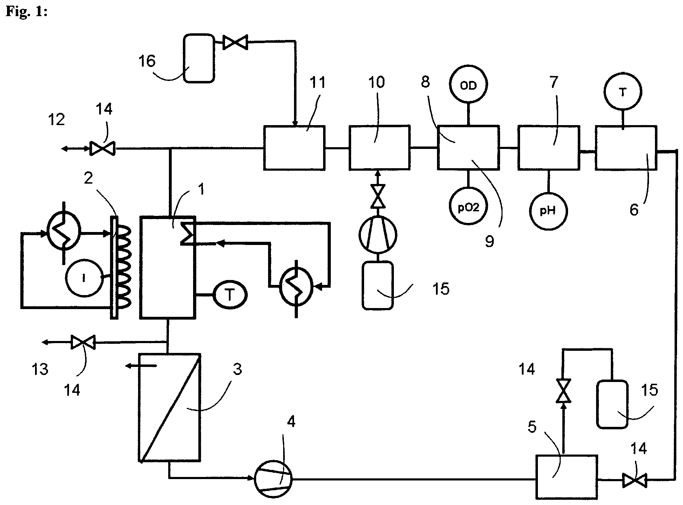

Eine beispielhafte Anlagenbeschreibung einer solchen Ausführungsform soll nachstehend anhand der Fig.1 erfolgen, wobei die Zahlenangaben nur als typische Werte verstanden werden sollen und keine Einschränkung darstellen.
Das Gesamtvolumen des Mediums betrug dabei 35 mL, das Reaktorvolumen insgesamt 20 mL, das Gesamtvolumen des Gefäßes betrug 10 mL. Die Kanäle waren 800 µm tief und 20 mm breit. Der Photoreaktor verfügte über Temperierkanäle und einen Temperatursensor und wurde mit zirkuliertem Wasser aus 23°C temperiert. Die Durchflussrate im Betrieb ist 10 ml/min, der Gaseintrag wurde automatisch in Abhängigkeit vom pH-Wert über Schaltventile gesteuert. Die eingesetzten Druck- und Temperatursensoren waren volumenminimiert. Die Optische Dichte wurde in einer Durchflusszelle aus Quarzglas über ein faseroptisches Mikrospektrometer erfasst. Als Pumpe diente eine Kreiselpumpe mit kleinem Innenvolumen.

Vorzugsweise umfasst die erfindungsgemäße Vorrichtung weiterhin einen Detektor zur Messung der Strahlungsintensität. Der Detektor erfasst entweder direkt die Strahlungsintensität oder eine damit zusammenhängende Größe. Als Detektor eignet sich beispielsweise eine Photodiode oder ein Phototransistor, die einfallende elektromagnetische Strahlung in ein elektrisches Signal umwandeln.

Der Detektor muss dabei so angebracht sein, dass eine möglichst repräsentative Detektion der Strahlung gewährleistet ist. Bevorzugt erfolgt die Anbringung daher seitlich an der transparenten Abdeckung, so dass Streulicht, das in der Abdeckung propagiert, detektiert wird, besonders bevorzugt aber direkt neben jeder einzelnen Strahlenquelle oder neben Gruppen von Strahlenquellen. Bei Leuchtdioden beispielsweise kann in jedem einzelnen Leuchtdiodengehäuse eine Photodiode untergebracht werden. Damit ist eine optimale Überwachung und Registrierung der Strahlungsintensität gewährleistet. Daneben gestattet auch die Messung des aktuellen Stromflusses über jede Leuchtdiode oder Gruppen von Leuchtdioden eine Überwachung der Strahlungsintensität, sofern Schwankungen der Strahlungsintensität infolge von Alterungsprozessen oder sonstiger Veränderungen der Leuchtdioden bekannt sind und berücksichtigt werden.

Weiterhin kann die Vorrichtung verschiedenene Sensoren zur pH-, Ionenkonzentration, Druck-, und Temperaturüberwachung sowie zur optischen Detektion von Lichtabsorption und/oder -streuung, und eine Prozessleiteinrichtung zur Automatisierung umfassen.

Die erfindungsgemäße Vorrichtung lässt sich für eine Vielzahl von photochemischen Prozessen einsetzen. Sie kann kontinuierlich, im Batch- oder Semi-Batch-Verfahren betrieben werden.

Überraschend wurde gefunden, dass die erfindungsgemäße Vorrichtung zur Kultivierung von Photosynthese-betreibenden Zellen und Mikroorganismen verwendet werden kann. Gegenstand der vorliegenden Erfindung ist daher auch die Verwendung der erfindungsgemäßen Vorrichtung zur Kultivierung von Photosynthese-betreibenden Zellen und/oder Mikroorganismen.

Insbesondere war es überraschend, dass die Kultivierung von Photosynthese-betreibenden Zellen und Mikroorganismen in der erfindungsgemäßen Vorrichtung reproduzierbar in einem Maßstab unter Bedingungen durchführbar ist, die den in technischen Großanlagen herrschenden Bedingungen sehr ähnlich sind. Hierdurch ist eine Übertragbarkeit der Ergebnisse aus der erfindungsgemäßen Vorrichtung auf einen größeren Produktionsmaßstab möglich. Dabei lassen sich beispielsweise Lichteintrag, Temperatur, Strömungsgeschwindigkeit, Gasaustausch und Nährstoffangebot in kleinem Maßstab verändern, um ohne die Verschwendung großer Materialmengen optimale Kultivierungsbedingungen zu identifizieren. Die erfindungsgemäße Vorrichtung erlaubt damit effektive Untersuchungen von optimalen Wachstumsbedingungen, die Stabilisierung von allen wichtigen Parametern wie Temperatur, pH-Wert, Gasaustausch, sie gestattet hohe Zellzahldichten und damit hohen Biomasseertrag und sie verwendet Bestrahlungseinheiten, die in Wellenlänge und Intensität variabel sind. Durch das Anlagenkonzept wird eine Skalierbarkeit der Ergebnisse erreicht.

Insbesondere erlaubt die erfindungsgemäße Verwendung der Vorrichtung zur Kultivierung von Photosynthese-betreibenden Zellen und Mikroorganismen Starterkulturen für Großanlagen zu erzeugen, wobei diese Starterkulturen besser an die Bedingungen der Großanlagen adaptiert sind als Kulturen aus z.B. Schüttelkolben. Die Starterkulturen dienen, falls erforderlich nach schrittweiser Vermehrung, zur Animpfung von Großanlagen.

Eine erfindungsgemäße Vorrichtung, die als Photobioreaktor zur Kultivierung von Photosyntesebetreibenden Zellen und/oder Mikroorganismen eingesetzt wird, umfasst mindestens eine Bestrahlungszone, eine Quelle für elektromagnetische Strahlung und Mittel zur Förderung eines Mediums durch die Vorrichtung.

Zur erfindungsgemäßen Verwendung wird der Photobioreaktor vorzugsweise kontinuierlich im Kreis betrieben, das heißt, das zu bestrahlende Medium mit den Zellen und/oder Mikroorganismen durchströmt den Photobioreaktor kontinuierlich im Kreis.

Die Bestrahlungszone ist vorzugsweise dadurch gekennzeichnet, dass das Volumen der Bestrahlungszone mindestens dem 0,5-fachen des Vorrichtungsvolumens entspricht. Damit verweilen die Zellen und/oder Mikroorganismen bei einem kontinuierlichen Kreisbetrieb mindestens dieselbe Zeit in den bestrahlten Zonen wie in den unbestrahlten Zonen.

Als Strahlenquelle werden bevorzugt Leuchtdioden eingesetzt, die vorzugsweise in einer oder zwei planaren Flächen angeordnet und zu der Bestrahlungszone parallel ausgerichtet sind.
Bevorzugt werden für die Kultivierung Photosyntese-betreibender Zellen und/oder Mikroorganismen Leuchtdioden verwendet, die Licht im Bereich des sichtbaren blauen und/oder roten Bereichs des Spektrums emittieren.

Die Zahl der Kanäle in der Bestrahlungszone beträgt vorzugsweise eins oder zwei. Bei mehr als zwei Kanälen kann es infolge der häufig bei Zellsuspensionen zu beobachtenden Ablagerungen zu unterschiedlichen Volumenströmen und damit unterschiedlichen Bestrahlungszeiten in einzelnen Kanälen kommen.

Die Mittel zur Förderung des Mediums durch den Photobioreaktor sollten eine möglichst geringe Scherrate aufweisen, da ansonsten eine Belastung auf die Zellen oder Mikroorganismen ausgeübt wird, die zur Verminderung der Produktivität durch gestresste und/oder abgestorbene Zellen oder Mikroorganismen und der Produktqualität durch Lyseprodukte führen kann. Bevorzugt werden Peristaltik-, Kolben-, Zahnrad- oder Kreiselpumpen eingesetzt, wobei diese vorzugsweise kleine Pumpenvolumina und niedrige Drehzahlen aufweisen. Untersuchungen haben gezeigt, dass das Kultivierungsergebnis durch eine automatisierte Steuerung der Drehzahl der Pumpe im Laufe der Kultivierung ebenso günstig beeinflusst werden kann, wie durch die gezielte Nachführung der Strahlungsintensität. Der optimale Ablauf einer Kultivierung wird nach einer Versuchsphase zur Ermittlung der optimalen Parameter durch die eingesetzte Automatisierungstechnik reproduzierbar eingehalten.

Vorzugsweise verfügt die Vorrichtung über eine Gaszu- und Gasabführung, um die Zellen oder Mikroorganismen mit gasförmigen Nährstoffen zu versorgen und gasförmige Stoffwechselprodukte zu entsorgen. Vorzugsweise erfolgt die Gaszuführung direkt im Einlass der Bestrahlungszone mit Hilfe einer Düse mit einem Durchmesser im Bereich von 10 µm bis 1000 µm, so dass kleine Gasblasen mit Durchmessern unter 1 mm durch die Bestrahlungszone des Photobioreaktors wandern. Dabei wird eine große Grenzfläche und damit ein effektiver Stoffaustausch zwischen Gas und Flüssigkeit ermöglicht, um beispielsweise den Eintrag von möglichst viel CO₂ in eine Zellsuspension und den Austrag des bei der Photosynthese gebildeten Sauerstoffs zu erlauben.

Die Anordnung mit Gaseindüsung kann auch zur Durchführung von photochemischen Umsetzungen unter Verwendung von Gasen wie z.B. Photohalogenierungen oder Photooxidationen eingesetzt werden.

Ein erfindungsgemäß verwendeter Photobioreaktor umfasst weiterhin vorzugsweise Sensoren zur pH-, Sauerstoff-, Temperatur-, Druck-, und optischen Überwachung, Pumpen- und Ventiltechnik, Rohrleitungen, Einrichtungen zur Datenerfassung und Prozessautomatisierung. Es ist zu beachten, dass der Anlagenteil, der i. A. nicht bestrahlt wird, ein möglichst kleines Flüssigkeitsvolumen aufweist.

Die Erfindung wird nachstehend anhand von Beispielen näher erläutert, ohne sie hierauf zu beschränken.

Es zeigen:
- **Fig. 1**: Prozessschema einer bevorzugten Ausführungsform der erfindungsgemäßen Vorrichtung zur Kultivierung Photosynthese-betreibender Zellen und/oder Mikroorganismen
- **Fig. 2**: Prozessschema einer bevorzugten Ausführungsform der erfindungsgemäßen Vorrichtung typischen Anlage zur Durchführung photochemischer Prozesse mit einem Mikromischer zur Vermischung reaktiver Spezies
- **Fig. 3.1**: Ausführungsbeispiel für die Kanalgestaltung: zwei parallel verlaufende, mäanderförmige Kanäle
- **Fig. 3.2**: Ausführungsbeispiel für die Kanalgestaltung: ein einziger mäanderförmiger Kanal
- **Fig. 3.3**: Ausführungsbeispiel für die Kanalgestaltung: eine spaltförmige Fläche, die von unten mit einer Flüssigkeitsverteilstruktur angeströmt wird

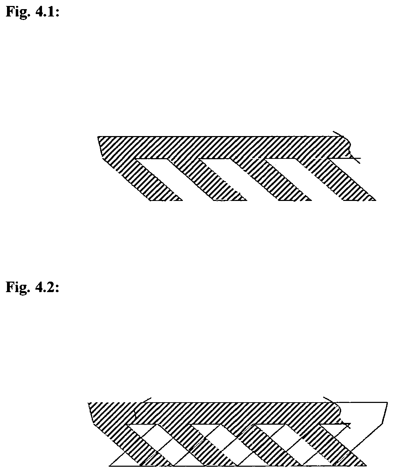

- **Fig. 4.1**: Strukturiertes Blech oder strukturierte Platte zur Erzeugung von dünnen bestrahlten Schichten
- **Fig. 4.2**: Anordnung von zwei strukturieren Blechen oder Platten, die gegensinnig gestapelt sind

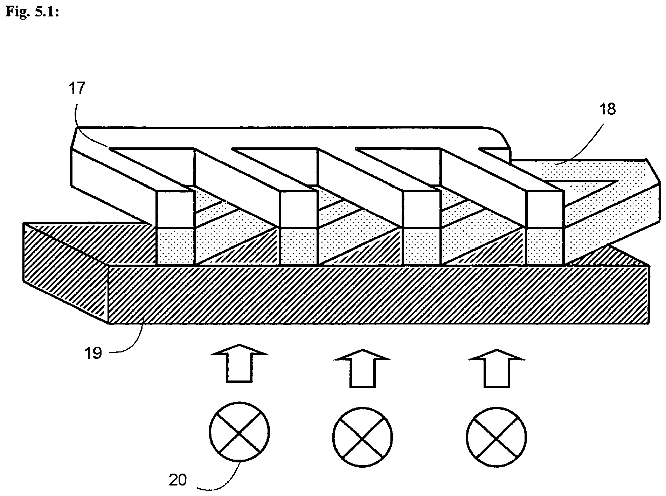

- **Fig. 5.1**: Schematische Darstellungen von Anordnungen der strukturierten gestapelten Platten mit transparenter Abdeckung und Strahlungsquellen, die von einer Seite bestrahlt werden.

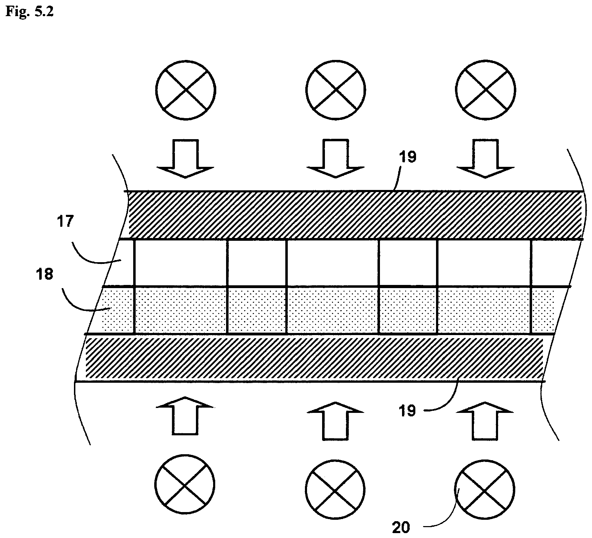

- **Fig. 5.2**: Schematische Darstellungen von Anordnungen der strukturierten gestapelten Platten mit transparenter Abdeckung und Strahlungsquellen, die von zwei Seiten bestrahlt werden.
- **Fig. 6.1**: Schematische Darstellung eines Teils der erfindungsgemäßen Vorrichtung mit Bestrahlungszone und einem Gaseinlass, der im Eingangsbereich der Bestrahlungszone angebracht ist, im Querschnitt von der Seite.
- **Fig. 6.2**: Schematische Darstellung eines Teils der erfindungsgemäßen Vorrichtung mit Bestrahlungszone und einem Gaseinlass, der im Eingangsbereich der Bestrahlungszone angebracht ist, im Querschnitt von der Seite.
- **Fig. 6.3**: Schematische Darstellung eines mäanderförmig verlaufenden Kanals mit Verjüngungen an den Umlenkstellen, in dem mit der Strömung des Mediums (gekennzeichnet durch die Pfeile) Gasblasen durch die Bestrahlungszone wandern.

In Figur 1 ist schematisch eine bevorzugte Ausführungsform der erfindungsgemäßen Vorrichtung zur Kultivierung von Photosynthese-betreibenden Zellen und/oder Mikroorganismen dargestellt. Die Vorrichtung umfasst eine Bestrahlungszone (1) und eine angeschlossene Peripherie mit Pumpen- und Ventiltechnik, verschiedene Sensoren zur pH (7)-, Druck-, Temperaturüberwachung (6)sowie zur optischen Detektion von Lichtabsorption (8) und -streuung, Temperiereinrichtungen, Rohrleitungen und Prozessleiteinrichtung zur Automatisierung. Neben dem Energieaustausch spielt der Stoffaustausch eine wichtige Größe, insbesondere der CO₂-Eintrag, der Sauerstoff-Austrag, die Nährstoffversorgung und ggf. die Abtrennung von giftigen Metaboliten.

Der Gaseintrag wird durch feine Düsen im Eingang der Bestrahlungszone (11), sowie Fig. 6.1, 6.2 erreicht. Feine Gasblasen mit Durchmessern unter 1 mm werden am Eingang der Bestrahlungszone erzeugt und wandern durch die Bestrahlungszone. Hierbei kommt es auf die Abstimmung der Strömungsgeschwindigkeit von Flüssig- und Gasphase an, um einen Transport der Gasblasen durch die Vorrichtung, insbesondere durch den Bestrahlungsraum zu gewährleisten und eine Koaleszenz zu vermindern. Koaleszenz führt zu vergrößerten Gasblasen, zu verminderten Grenzflächen zwischen Gas und Flüssigkeit und damit zu verschlechtertem Stoffaustausch.

Hinter dem Ausgang der Bestrahlungszone befindet sich ein Ausgleichsgefäß (3), das zur Abtrennung von Gas- und Flüssigkeit dient. Das hier abgetrennte Gas kann auch zur erneuten Eindüsung verwendet werden, solange der Sauerstoffgehalt nicht zu hoch ist.

Die Ventiltechnik der Eindüsung des CO₂-haltigen Gases bildet zusammen mit dem pH-Sensor und der Einrichtung zur Datenerfassung und Steuerung eine Regelungseinheit, mit deren Hilfe der pH-Wert in der umgepumpten Suspension konstant gehalten wird. Eine weitere Eindüsungsstelle dient zur optionalen Zugabe von Nährstofflösung in Abhängigkeit von der Wachstumsphase oder registrierten Parametern. Eine optische Durchflusszelle dient zur Detektion von optischen Parametern wie Absorption, Lichtstreueigenschaften oder Fluoreszenz. Es hat sich als vorteilhaft erwiesen, wenn alle Komponenten mit möglichst niedrigem Volumen und kraft- oder formschlüssigen Verbindungen miteinander verbunden werden. Dadurch können die Komponenten leicht herausgenommen, ausgewechselt oder an anderer Stelle im Prozessschema eingesetzt werden, was im Rahmen von Untersuchungen oder Optimierungsaufgaben einen Zeitvorteil bei Umbau- oder Reinigungsarbeiten bietet.

Weitere Sensoren können neben Temperatur, die vorzugsweise im Reaktorraum, im Puffergefäß und der Pumpe eingeregelt wird, auch den Sauerstoffpartialdruck elektrochemisch oder faseroptisch mit Hilfe sogenannter Lumineszenzoptoden erfassen. Ein optischer Detektor erfasst die optische Dichte, die mit der Biomasse korreliert. Sinnvoll ist auch die Ankopplung eines faseroptischen Spektrometers, so dass spektral aufgelöste Messung von Absorptions- und Lichtstreuung möglich sind. Dadurch kann zum einen das Wachstum online verfolgt, aber auch mit Hilfe von absorptionsspektroskopischen Messungen der Farbstoffgehalt geprüft werden.

Es hat sich als vorteilhaft herausgestellt, die Kulturen dann zu ernten, wenn eine bestimmte Zellzahldichte oder Farbstoffkonzentration erreicht und vorzugsweise durch die eingesetzten Sensoren angezeigt wurde.

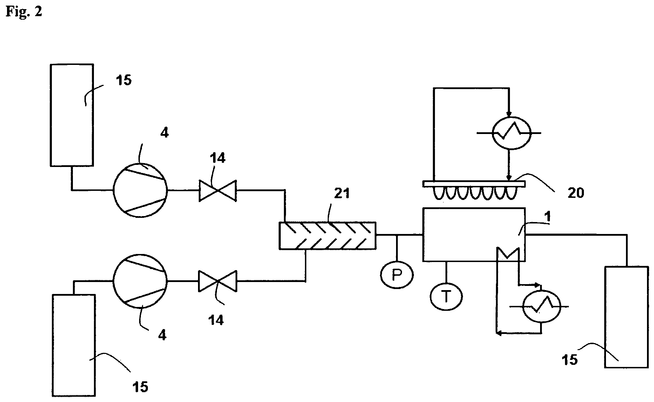

Figur 2 zeigt das Prozessschema einer bevorzugten Ausführungsform der erfindungsgemäßen Vorrichtung zur Durchführung photochemischer Reaktionen, wobei der eigentlichen photochemischen Umsetzung in der Bestrahlungszone ein Mikromischer zur Dosierung von Flüssigkeiten wie z.B. Reaktionskomponenten vorgeschaltet sein kann.
Die einzelnen Funktionalitäten der Vorrichtungen in den Figuren 1 und 2 sind vorzugsweise in Modulbauweise ausgeführt, so dass Modifikationen des Anlagenschemas leicht und schnell möglich sind. Besonders bevorzugt ist der Einsatz einer kraft- oder formschlüssigen Verbindung ohne Rohrleitungen oder sonstige Verbindungstechnik zwischen den einzelnen Modulen, um das Anlagenvolumen zu minimieren.

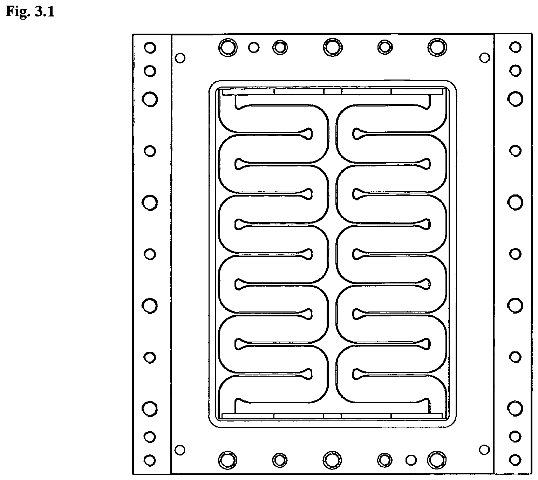

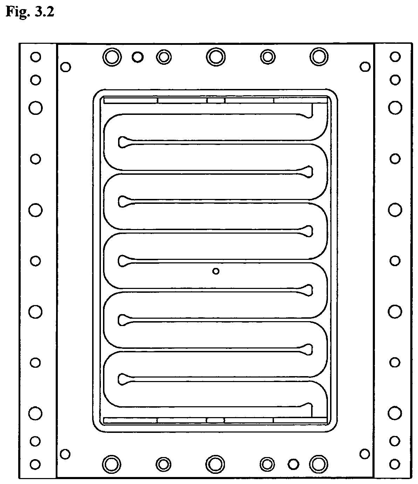

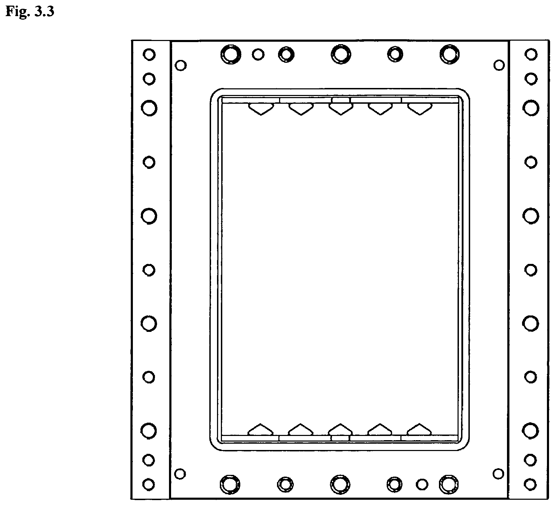

Eine typische Ausführung der Kanalgestaltung in der Bestrahlungszone zeigt Fig. 3.1. Das Medium wird in zwei Kanälen mäanderförmig vor der Strahlungsquelle geführt, wobei bei der Verwendung von mehreren Leuchtdioden die Position und der Abstand dieser so gewählt sind, dass die Kanäle vollständig ausgeleuchtet werden. Die Aufstellung der Bestrahlungszone erfolgt vorzugsweise vertikal, so dass die Kanäle von unten nach oben entgegen der Schwerkraft durchströmt werden. Weitere sinnvolle praktische Kanalgestaltungen sind in den Figuren 3.2 und 3.3 dargestellt. Dort wird entweder nur ein einzelner Kanal zur Fluidführung (Fig. 3.2) oder aber ein einziger großer Spalt mit einer geeigneten Fluidverteilung am Eintritt in die Bestrahlungszone eingesetzt (Fig. 3.3).

In einer bevorzugten Ausführungsform werden Strömungseinlagen verwendet, um die dünne bestrahlte Schicht zu erzeugen. Die Strömungseinlagen bestehen vorzugsweise aus strukturierten Blechen oder Platten, die Stapelweise in den Kanal eingelegt werden. Dadurch müssen in der Bestrahlungszone (Reaktorzonenplatte) selbst keine feinen Strukturen eingearbeitet werden. Die Anordnung aus Kanalstrukturen und Strömungseinlagen ist damit zerlegbar, variabel gestaltbar und lässt sich leichter reinigen.

In den Figuren 6.1 und 6.2 ist ein Teil der erfindungsgemäßen Vorrichtung dargestellt. Dieser Teil umfasst einen Gaseinlass im Eingangsbereich der Bestrahlungszone. Die Figuren 6.1 und 6.2 zeigen verschiedene Ausführungsformen des Gaseinlasses. Durch eine Düse werden kleine Gasblasen in das Medium eingebracht (dargestellt durch den gestrichelten Pfeil und die kleinen Kreise), die durch die Strömung (dargestellt durch die dünnen durchgezogenen Pfeil) mit dem Medium mitgetragen werden. Das Medium durchströmt die bevorzugt vertikal ausgerichtete Bestrahlungszone vorzugsweise von unten nach oben. Die Bestrahlung ist durch den dicken Pfeil gekennzeichnet. Die Strahlungsquelle ist wie allgemein üblich durch einen Kreis mit zwei sich kreuzenden Linien dargestellt.

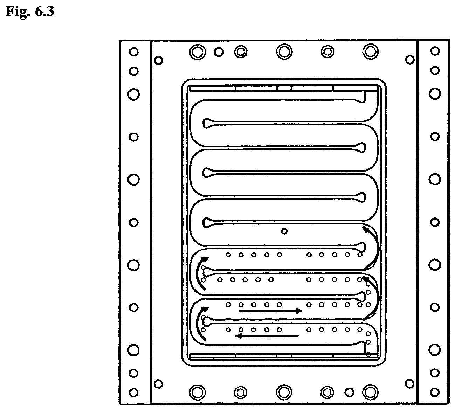

Figur 6.3 zeigt die Anordnung aus den Figuren 6.1 und 6.2 in einer Aufsicht. Die Bestrahlungszone wird aus Sicht des Betrachters bestrahlt. Sie umfasst einen mäanderförmig durch die Bestrahlungszone verlaufenden Kanal. Gasblasen, die im Eingangsbereich der Bestrahlungszone eingebracht wurden, durchwandern zusammen mit dem Medium die Bestrahlungszone (dargestellt durch die Pfeile).

### Bezugszeichen

- 1: Bestrahlungszone mit Fluidführung, Thermostatisierflüssigkeit, Temperatursensor
- 2: Bestrahlungseinheit mit Thermostatisierflüssigkeit und Intensitätsmessung
- 3: Ausgleichsgefäß mit Gasabscheidung
- 4: Pumpe
- 5: Filtrationseinheit
- 6: Temperatursensor
- 7: pH-Sensor
- 8: optischer Sensor für Transmission
- 9: Sauerstoffsensor
- 10: Nährstoffzugabe
- 11: Gaseindüsung
- 12: Entnahme- und Befüllungsöffnung
- 13: Entleerungsöffnung
- 14: Absperrventil
- 15: Flüssigkeitsbehälter
- 16: Gasbehälter
- 17: strukturiertes Blech oder Platte
- 18: strukturiertes Blech oder Platte
- 19: Abdeckung
- 20: Strahlungsquelle
- 21: Mikromischer

## Patentansprüche

1. Vorrichtung zur Durchführung photochemischer und photobiotechnologischer Prozesse mindestens umfassend eine mikrostrukturierte Bestrahlungszone, eine Quelle für elektromagnetische Strahlung und Mittel zur Förderung eines Mediums durch die Vorrichtung, wobei das Volumen der Bestrahlungszone mindestens dem 0,5-fachen des Vorrichtungsvolumens entspricht und die Bestrahlungszone einen oder mehrere Kanäle umfasst, die die Bestrahlungszone gerade oder mäanderförmig durchlaufen und im Querschnitt rechteckig oder halbrund ausgeführt sind und eine Tiefe im Bereich von 10 µm bis 2000 µm aufweisen, **dadurch gekennzeichnet, dass** zur Ausbildung einer oder mehrerer dünner Schichten des Mediums im Bestrahlungsraum Schichten aus strukturierten Blechen oder Platten eingebracht sind.

2. Vorrichtung nach Anspruch 1, **dadurch gekennzeichnet dass** die Kanäle eine Tiefe im Bereich von 500 µm bis 1000 µm aufweisen.

3. Vorrichtung. nach Anspruch 1 oder 2, **dadurch gekennzeichnet dass** die Kanäle eine Breite im Bereich von 10 mm bis 50 mm aufweisen

4. Vorrichtung nach einem der Ansprüche 1 bis 3, **dadurch gekennzeichnet dass** die Kanäle eine Breite im Bereich von 15 mm bis 40 mm aufweisen.

5. Vorrichtung nach einem der Ansprüche 1 bis 4, **dadurch gekennzeichnet, dass** die mikrostrukturierte Bestrahlungszone mit einem in Schwerkraftrichtung unten angebrachten versehenem Medieneinlass und einem in Schwerkraftrichtung oben angebrachtem Medienauslass ausgestattet ist.

6. Vorrichtung nach einem der Ansprüche 1 bis 5, **dadurch gekennzeichnet, dass** der in Schwerkraftrichtung unten angebrachten versehenem Medieneinlass mit einer Gaseinspeisung versehen ist.

7. Vorrichtung nach einem der Ansprüche 1 bis 6, **dadurch gekennzeichnet, dass** die Kanäle an ihren Umlenkstellen Verjüngungen aufweisen.

8. Vorrichtung nach einem der Ansprüche 1 bis 7, **dadurch gekennzeichnet, dass** die Strahlungsquelle eine Anordnung von Leuchtdioden in einer planaren Fläche ist, die parallel zur Bestrahlungszone angeordnet ist.

9. Vorrichtung nach einem der Ansprüche 1 bis 8, **dadurch gekennzeichnet, dass** die Bestrahlungszone zwischen zwei planaren Anordnungen von Leuchtdioden eingebracht ist, wobei die Anordnungen von Leuchtdioden und die Bestrahlungszone jeweils parallel zueinander ausgerichtet sind.

10. Vorrichtung nach einem der Ansprüche 1 bis 9, **dadurch gekennzeichnet, dass** das Mittel zur Förderung eines Mediums durch die Vorrichtung eine Peristaltik-, Kolben-, Zahnrad-, Membran- oder Kreiselpumpe ist.

11. Verwendung einer Vorrichtung gemäß einem der Ansprüche 1 bis 10 zur Kultivierung von Photosynthese-betreibenden Zellen und/oder Mikroorganismen.

12. Verwendung nach Anspruch 11, **dadurch gekennzeichnet, dass** in der Bestrahlungszone Gasblasen mit einem Durchmesser kleiner als 1 mm erzeugt werden, so dass die Gasblasen mit dem Medium durch die Bestrahlungszone wandern.

13. Verwendung nach einem der Ansprüche 11 oder 12, **dadurch gekennzeichnet, dass** die Bestrahlungszone vertikal ausgerichtet ist und das Medium die Bestrahlungszone entgegen der Richtung der Schwerkraft von unten nach oben durchströmt.

14. Verwendung nach einem der Ansprüche 11 bis 13 zur Erzeugung von Starterkulturen für eine größere Anlage.

## Claims

1. Device for carrying out photochemical and photobiotechnological processes, comprising at least a microstructured irradiation zone, a source of electromagnetic radiation and means for transporting a medium through the device, the volume of the irradiation zone corresponding to at least 0.5 times the device volume, and the irradiation zone comprising one or more channels which pass through the irradiation zone in a linear or meander-shaped manner and are constructed so as to be rectangular or semicircular in cross section and have a depth in the range from 10 µm to 2000 µm, **characterized in that**, for development of one or more thin layers of the medium in the irradiation chamber, layers of structured metal sheets or plates are introduced.

2. Device according to Claim 1, **characterized in that** the channels have a depth in the range from 500 µm to 1000 µm.

3. Device according to Claim 1 or 2, **characterized in that** the channels have a width in the range from 10 mm to 50 mm.

4. Device according to any one of Claims 1 to 3, **characterized in that** the channels have a width in the range from 15 mm to 40 mm.

5. Device according to any one of Claims 1 to 4, **characterized in that** the microstructured irradiation zone is equipped with a media inlet that is provided mounted at the bottom in the direction of gravity and a media outlet that is mounted at the top in the direction of gravity.

6. Device according to any one of Claims 1 to 5, **characterized in that** the media inlet that is provided mounted at the bottom in the direction of gravity is provided with a gas feed.

7. Device according to any one of Claims 1 to 6, **characterized in that** the channels have taperings at their deflection points.

8. Device according to any one of Claims 1 to 7, **characterized in that** the radiation source is an arrangement of light-emitting diodes in a planar surface that is arranged in parallel to the irradiation zone.

9. Device according to any one of Claims 1 to 8, **characterized in that** the irradiation zone is mounted between two planar arrangements of light-emitting diodes, wherein the arrangements of light-emitting diodes and the irradiation zone are in each case orientated in parallel to one another.

10. Device according to any one of Claims 1 to 9, **characterized in that** the means for transporting a medium through the device is a peristaltic pump, piston pump, gear pump, diaphragm pump or centrifugal pump.

11. Use of a device according to any one of Claims 1 to 10 for culturing photosynthesizing cells and/or microorganisms.

12. Use according to Claim 11, **characterized in that**, in the irradiation zone, gas bubbles having a diameter of less than 1 mm are generated, in such a manner that the gas bubbles migrate through the irradiation zone together with the medium.

13. Use according to either of Claims 11 or 12, **characterized in that** the irradiation zone is orientated vertically, and the medium flows through the irradiation zone from bottom to top against the direction of gravity.

14. Use according to any one of Claims 11 to 13 for generating starter cultures for a relatively large plant.

## Revendications

1. Dispositif destiné à réaliser des processus photochimiques et photobiotechnologiques, comprenant au moins une zone d'irradiation microstructurée, une source d'un rayonnement électromagnétique et des moyens pour transporter un milieu à travers le dispositif, le volume de la zone d'irradiation correspondant au moins à 0,5 fois le volume du dispositif et la zone d'irradiation comprenant un ou plusieurs canaux qui traversent la zone d'irradiation en ligne droite ou en forme de méandres et qui sont réalisés avec une section transversale rectangulaire ou semi-circulaire et qui présentent une profondeur de l'ordre de 10 µm à 2000 µm, **caractérisé en ce que** pour créer une ou plusieurs couches minces du milieu dans l'espace d'irradiation, des couches de tôles ou plaques structurées sont introduites dans l'espace d'irradiation.

2. Dispositif selon la revendication 1, **caractérisé en ce que** les canaux présentent une profondeur de l'ordre de 500 µm à 1000 µm.

3. Dispositif selon la revendication 1 ou la revendication 2, **caractérisé en ce que** les canaux ont une largeur de l'ordre de 10 mm à 50 mm.

4. Dispositif selon l'une quelconque des revendications 1 à 3, **caractérisé en ce que** les canaux ont une largeur de l'ordre de 15 mm à 40 mm.

5. Dispositif selon l'une quelconque des revendications 1 à 4, **caractérisé en ce que** la zone d'irradiation microstructurée est équipée d'une entrée garnie pour le milieu, montée sur le dessous dans la direction de la gravité et une sortie pour le milieu, montée sur le dessus dans la direction de la gravité.

6. Dispositif selon l'une quelconque des revendications 1 à 5, **caractérisé en ce que** l'entrée garnie pour le milieu, montée sur le dessous dans la direction de la gravité est garnie d'une alimentation de gaz.

7. Dispositif selon l'une quelconque des revendications 1 à 6, **caractérisé en ce que** sur leurs points de renvoi, les canaux comportent des rétrécissements.

8. Dispositif selon l'une quelconque des revendications 1 à 7, **caractérisé en ce que** la source de rayonnement est un agencement de diodes électroluminescentes dans une surface planaire qui est placé à la parallèle de la zone d'irradiation.

9. Dispositif selon l'une quelconque des revendications 1 à 8, **caractérisé en ce que** la zone d'irradiation est insérée entre deux agencements planaires de diodes électroluminescentes, les agencements de diodes électroluminescentes et la zone de rayonnement étant chaque fois orientés parallèlement l'un par rapport à l'autre.

10. Dispositif selon l'une quelconque des revendications 1 à 9, **caractérisé en ce que** le moyen destiné à transporter un milieu à travers le dispositif est une pompe péristaltique, une pompe à piston, une pompe à engrenages, une pompe à membrane ou une pompe centrifuge.

11. Utilisation d'un dispositif selon l'une quelconque des revendications 1 à 10 pour la culture de cellules et/ou microorganismes assurant la photosynthèse.

12. Utilisation selon la revendication 11, **caractérisée en ce que** dans la zone d'irradiation sont créées des bulles de gaz d'un diamètre inférieur à 1 mm, de sorte que les bulles de gaz migrent avec le milieu à travers la zone d'irradiation.

13. Utilisation selon l'une quelconque des revendications 11 ou 12, **caractérisée en ce que** la zone d'irradiation est orientée à la verticale et **en ce que** le milieu irrigue la zone d'irradiation à l'encontre de la direction de la gravité, du bas vers le haut.

14. Utilisation selon l'une quelconque des revendications 11 à 13 pour générer une culture de démarrage pour une installation d'assez grande envergure.
